Europäisches Patentamt

European Patent Office

Office européen des brevets

(11) Veröffentlichungsnummer: **0 297 295 B1**

## (12) EUROPÄISCHE PATENTSCHRIFT

(45) Veröffentlichungstag der Patentschrift: **28.10.92**

(21) Anmeldenummer: **88108659.9**

(22) Anmeldetag: **31.05.88**

Die Akte enthält technische Angaben, die nach dem Eingang der Anmeldung eingereicht wurden und die nicht in dieser Patentschrift enthalten sind.

(51) Int. Cl.$^5$: **C07C 209/24**, C07C 211/03, C07C 211/48, C07C 217/08, C07D 295/03

(54) **Verfahren zur Herstellung von Methylaminen.**

(30) Priorität: **30.06.87 DE 3721539**

(43) Veröffentlichungstag der Anmeldung:
**04.01.89 Patentblatt 89/01**

(45) Bekanntmachung des Hinweises auf die Patenterteilung:
**28.10.92 Patentblatt 92/44**

(84) Benannte Vertragsstaaten:
**BE DE FR GB IT NL**

(56) Entgegenhaltungen:
**EP-A- 0 142 868
DE-A- 1 932 422
DE-A- 2 618 580
DE-A- 3 544 510
US-A- 3 136 819**

(73) Patentinhaber: **HOECHST AKTIENGESELL-SCHAFT
Postfach 80 03 20
W-6230 Frankfurt am Main 80(DE)**

(72) Erfinder: **Weber, Jürgen, Dr., Dipl.-Chem.
Bunsenstrasse 17
W-4200 Oberhausen 11(DE)**
Erfinder: **Kampmann, Detlef, Dr., Dipl.-Chem.
Rankenweg 3
W-4630 Bochum(DE)**
Erfinder: **Kniep, Claus
Rosenstrasse 93
W-4200 Oberhausen 1(DE)**

**Beschreibung**

Die vorliegende Erfindung betrifft ein Verfahren zur Herstellung von Methylaminen durch Umsetzung von Aminen mit Formaldehyd und Wasserstoff an einem festangeordneten Katalysator in flüssiger Phase. Als Amine kommen einwertige oder mehrwertige primäre oder sekundäre Amine sowie Mischungen derselben in Betracht. Die Reaktion verläuft bei erhöhter Temperatur und erfordert in der Regel einen erhöhten Druck. Je nach den gewählten Reaktionsbedingungen führt sie zu einem teilweisen oder vollständigen Ersatz der am Aminstickstoffatom befindlichen Wasserstoffatome, die unter Bildung von Wasser gegen eine Methylgruppe ausgetauscht werden. Man bezeichnet diese Umsetzung daher auch als hydrierende N-Methylierung von Aminen.

Tertiäre Amine stellen technisch bedeutsame Verbindungen dar. Sie können als Polymerisations- und Härtungskatalysatoren für die Herstellung von Kunststoffen auf Epoxid- und Urethanbasis dienen. Ferner sind sie als Korrosionsinhibitoren und Adsorptionsmittel für die Synthesegaswäsche geeignet. Das trifft besonders auf die leicht herstellbaren Methyl- und Dimethylderivate zu.

Die hydrierende Methylierung von Aminen mit Formaldehyd und Wasserstoff ist ein wichtiger Syntheseweg zur Herstellung methylierter Amine. Eine zusammenfassende Übersicht bezüglich dieses Verfahrens findet sich in Houben-Weyl, Methoden der organischen Chemie; Band XI/1, Seiten 641 bis 647, 4. Auflage (1957).

Eine Übertragung diskontinuierlich durchgeführter Versuche auf eine kontinuierliche Umsetzung von Aminen mit Formaldehyd und Wasserstoff ist nicht möglich. Hierfür sind verschiedene Grunde verantwortlich.

Bei der kontinuierlichen N-Methylierung kann man druckfeste Rohrreaktoren verwenden, die den Hydrierkatalysator in stückiger Form enthalten. Die Ausgangsstoffe oder deren Umsetzungsgemische werden dem Rohrreaktor entweder am Kopf oder am Boden zugeführt. Je nach Zugabeart spricht man von Riesel- oder Sumpffahrweise. Bei längerem Gebrauch zerfallen die Hydrierkatalysatoren in zunehmendem Maße. Die resultierenden feinteiligen Partikel führen entweder in den Rohrreaktoren oder in nachgeschalteten Anlageteilen zu Verlegungen. Dadurch kommt es zu einem Druckanstieg in der Reaktionsapparatur, mit der zwangsweisen Folge, daß die Umsetzung unterbrochen werden muß, um die Verstopfungen zu beseitigen.

Unerwünscht ist ferner die Entstehung von Ameisensäure, die sich vermutlich durch Cannizzaro-Reaktion aus Formaldehyd bildet. Sie entzieht der Reaktion eine entsprechende Menge Amin als Salz. Die freie Säure wie auch das Aminsalz begünstigen Korrosionen im Reaktorsystem.

Weitere, eine kontinuierliche Umsetzung störende Nebenreaktionen beruhen auf der Polymerisation von Formaldehyd und der Polykondensation zwischen Amin und Formaldehyd zu Hexahydrotriazinen oder im Falle mehrwertiger Amine zu höhermolekularen Verbindungen. Die Bildung polymerer Stoffe führt zu Verklebungen der Katalysatorfüllung und somit zu Blockierung des Reaktors. Ein Kontaktwechsel ist die unvermeidliche Folge.

Diese bekannten Nachteile werden auf unterschiedliche Weise umgangen.

Zur Vermeidung frühzeitigen Kontaktzerfalls empfiehlt die DE-A 25 45 695 die Verwendung eines vorbehandelten Kobalt-oder Nickelkatalysators. Die Vorbehandlung besteht darin, daß dieser Katalysator für eine gewöhnliche Hydrierung oder Aminierung verwendet wird, ehe er für die hydrierende N-Methylierung eingesetzt wird.

Die DE-A 26 18 580 lehrt den Gebrauch eines speziellen Katalysators, der wenigstens 60 % Kobalt, 10 bis 30 % Kupfer enthalt und im wesentlichen frei von Trägern ist. Das Reaktionsgemisch wird in einem Flüssigkeitskreislauf durch den Hydrierreakor geleitet, wobei eine Reaktionstemperatur von 110°C nicht überschritten werden soll. Die Ausgangsstoffe Amin und Formaldehyd werden dem Flüssigkeitskreislauf außerhalb des Reaktors zugesetzt, um Nebenreaktionen zu vermeiden.

Die EP-A 0 142 868 empfiehlt für die N-Methylierung von Aminen die Verwendung von Hydrierkatalysatoren auf Aktivkohle. Trägerkatalysatoren auf Basis Aluminiumoxid, Siliciumdioxid und Kieselerde sind wegen der schlechten Verteilung des Katalysators unerwünscht. Trägerfreie Metallkatalysatoren sind zwar sehr aktiv, liefern aber keine guten Ergebnisse.

Wie dem vorstehend genannten Stand der Technik zu entnehmen ist, mangelte es nicht an Versuchen, die bei der Umsetzung von Aminen mit Formaldehyd und Wasserstoff auftretenden Schwierigkeiten durch Verwendung besonderer Katalysatoren zu vermeiden.

Daher besteht ein Bedarf nach einem Verfahren, das sowohl einfach auszuüben ist als auch den Gebrauch üblicher Hydrierkatalysatoren für die N-Methylierung ermöglicht. Es soll nicht nur bei hoher Selektivität hohe Ausbeuten liefern, sondern auch allgemein, d.h. unabhängig von der Art des Amins, anwendbar sein.

2

EP 0 297 295 B1

Diese Aufgaben werden gelöst durch ein Verfahren zur Herstellung von Methylaminen durch Umsetzung von Aminen, Formaldehyd und Wasserstoff als Ausgangsstoffe unter Druck und erhöhter Temperatur an einem festangeordneten Katalysator in flüssiger Phase. Es ist dadurch gekennzeichnet, daß der Formaldehyd 0 bis 50 Gew.% wasser enthält, und, die Ausgangsstoffe voneinander getrennt auf eine vorgegebene Temperatur erhitzt und anschließend in Gegenwart des festangeordneten Katalysators unter Vermischung zusammengeführt werden.

Die Ausgangsstoffe werden bei 0,1 bis 30, insbesondere 1 bis 20, bevorzugt 2 bis 15 MPa umgesetzt. Die Reaktionstemperatur beträgt 20 bis 250, insbesondere 50 bis 200, bevorzugt 70 bis 150°C.

Das erfindungsgemäße Verfahren kann diskontinuierlich und kontinuierlich betrieben werden. Es ist besonders für einen kontinuierlichen Prozeß geeignet.

Als festangeordnete Katalysatoren kommen übliche Hydrierkontakte in Betracht. Druck und Temperatur sind in gewissem Umfange auch von der Art des Katalysators abhängig und müssen entsprechend aufeinander abgestimmt werden.

Die Umsetzung kann sowohl im geraden Durchgang als auch unter Zuhilfenahme eines Reaktionsproduktkreislaufes durchgeführt werden. Die Ausgangsstoffe können dem Reaktor, in dem der Katalysator untergebracht ist, am Kopf oder am Boden zugeleitet werden. Nach einer besonderen Ausführungsform werden die Einsatzstoffe dem Reaktor am Boden zugeführt und die Umsetzung erfolgt im geraden Durchgang, d.h. ohne Kreislauf des Reaktionsproduktes. Das Reaktionsgemisch verläßt den Reaktor am Kopf.

Das erfindungsgemäße Verfahren ist unabhängig von der Art der verwendeten Amine. Es läßt sich allgemein auf sämtliche organische Verbindungen mit einer oder mehreren primären oder sekundären Amingruppen anwenden. Als Amine lassen sich einwertige und/oder mehrwertige primäre und/oder sekundäre Amine verarbeiten. Aliphatische oder cycloaliphatische Amine können ebenso in die erfindungsgemäße N-Methylierung eingesetzt werden wie araliphatische, aromatische oder heterocyclische Amine. Auch Mischungen derselben lassen sich verarbeiten.

Die Amine besitzen insgesamt 1 bis 40 Kohlenstoffatome. Die vorhandenen Substituenten können geradkettige und/oder verzweigte Alkylreste mit 1 bis 20 Kohlenstoffatomen, unsubstituierte und/oder substituierte Cycloalkylreste mit 5 bis 20 Kohlenstoffatomen, unsubstituierte und/oder substituierte aromatische Gruppen mit 6 bis 20 Kohlenstoffatomen oder heterocyclische Reste mit 4 bis 20 Kohlenstoffatomen, die als Heteroatom Sauerstoff, Schwefel und/oder Stickstoff enthalten können, sein.

Die Amine können gleiche oder verschiedene Substituenten der vorstehend genannten Art besitzen.

Beispiele für aliphatische Amine sind: Methylamin, Ethylamin, Propylamin, n- und i-Butylamin, Pentylamin, Hexylamin, 2-Ethylhexylamin, Octylamin, Dodecylamin, Stearylamin, Dimethylamin, Diethylamin, Dipropylamin, Dibutylamin, Dipentylamin, Dihexylamin, Di-2-ethylhexylamin, Dioctylamin, Ethylendiamin, 1,3-Diaminopropan, 1,4-Diaminobutan, 1,6-Diaminohexan (Hexamethylendiamin-(1,6)), Polyalkylenpolyamine, Hexamethylentetramin, Ethanolamin, Propanolamin, Diethanolamin.

Als Beispiele für cycloaliphatische Amine seien genannt: Cyclopentylamin, Cyclohexylamin und Tricyclodecanamine. Die Ringsysteme können Substituenten, insbesondere Alkylreste, aufweisen.

Beispiele für araliphatische Amine sind: Benzylamin, alpha-und beta-Phenylethylamin, N-Methylbenzylamin und Dibenzylamin.

Beispiele für aromatische Amine sind: Anilin, Toluidin, N-alkylierte Aniline und Toluidine, Benzidin (4.4'-Diaminodiphenyl), Phenylendiamine, Diphenylamin, substituierte und unsubstituierte Naphtylamine.

Beispiele für heterocyclische Amine sind: Piperidin, Piperazin, Pyrrol und Diazole.

Wesentlich für das Gelingen des erfindungsgemäßen Verfahrens ist, daß das im Reaktor vorliegende Gemisch einen begrenzten Wassergehalt aufweist.

Zu hohe Wasseranteile sind unerwünscht, da sie den festangeordneten Katalysator schädigen. Einerseits beeinträchtigen sie die Aktivität des Katalysators und verringern sowohl Umsatz als auch Selektivität der Reaktion, andererseits führen sie zu einem raschen Zerfall des festangeordneten Katalysators.

Die Begrenzung des Wassergehaltes erreicht man, indem man Ausgangsstoffe mit vermindertem Wassergehalt verwendet. In der Regel weist das Amin Wasser nur in untergeordneten Mengen oder in Spuren auf. Dagegen sind in dem technisch gebräuchlichem Formaldehyd, der etwa 60 Gew.-% und mehr Wasser aufweisen kann, betrachtliche Wasseranteile enthalten. Zu diesem Wasser, das mit den Ausgangsstoffen in die Reaktion eingebracht wird, kommt noch das durch die Umsetzung freigesetzte Reaktionswasser hinzu.

Die Summe beider Wasseranteile soll, bezogen auf das den festangeordneten Katalysator umströmende Flüssigkeits-Gas-Gemisch einen Wert von 50 Gew.-% nicht übersteigen.

Besonders zweckmäßig ist es, den Wassergehalt der Formaldehydlösung zu begrenzen. Er soll einen Wert von 50 Gew.-% bezogen auf Formaldehydlosung nicht übersteigen. Geeignet sind Formaldehydlösungen mit 0 bis 50 Gew.-% Wasser. Besonders bewährt haben sich solche mit 5 bis 30 Gew.-% Wasser. Bevorzugt wird ein Formaldehyd mit 7 bis 15 Gew.%-Wasser. Mit gutem Erfolg kann auch Paraformaldehyd, z.B. in suspendierter Form, eingesetzt werden.

Unabhängig von der mit den Ausgangsstoffen der Reaktion zugeführten Wassermenge erhöht sich der Wassergehalt im Reaktionsgemisch infolge der Bildung von Reaktionswasser. Diese Steigerung des Wasseranteils kann dazu führen, daß sich ein heterogenes, flüssiges Produktgemisch, das aus organischen und aus wäßrigen Anteilen besteht, ausbildet. Die resultierende Wasserphase lagert sich unten im Reaktor ab und verhindert die Umsetzung in dem Bereich, wo sie den festangeordneten Katalysator bedeckt.

Um die Bildung einer unerwunschten Wasserphase zu vermeiden, empfiehlt sich der Zusatz eines sowohl Wasser als auch organische Verbindungen lösenden Solvens. Geeignete Solventien sind sowohl aliphatische Alkohole mit 1 bis 5 Kohlenstoffatomen, Ether wie Tetrahydrofuran und Dioxan, sowie Dimethylsulfoxid als auch Mischungen der vorstehend genannten Stoffe. Das Solvens kann sowohl dem Amin als auch dem Formaldehyd zugefügt werden.

Entscheidend für das erfindungsgemäße Verfahren ist ferner, daß die Ausgangsstoffe voneinander getrennt auf eine vorgegebene Temperatur aufgeheizt werden. Dies bedeutet, daß man Amin, Formaldehyd und Wasserstoff über eigene Leitungen fuhrt und erwärmt, bevor man die Reaktion durchführt.

Man kann auch den Wasserstoff im Gemisch mit Amin oder im Gemisch mit Formaldehyd unter Vorerwärmen der Reaktion zuleiten. Es ist ferner möglich, den Wasserstoff auf Amin und Formaldehyd zu verteilen. Nach einer besonderen Ausführungsform wird Wasserstoff mit Amin vermischt und erwärmt. Hingegen ist es nicht zulässig, Amin und Formaldehyd zu mischen und zu erwärmen, ehe die Reaktion erfolgt. Amin und Formaldehyd sind stets voneinander getrennnt zu führen und dabei auf eine vorgegebene Temperatur aufzuheizen.

Die vorgegebene Temperatur richtet sich im wesentlichen nach der Reaktionstemperatur. Sie sollte nicht mehr als 20, insbesondere 10, bevorzugt 5°C unterhalb der Reaktionstemperatur und nicht mehr als 10, insbesondere 5, bevorzugt 0°C oberhalb der Reaktionstemperatur liegen. Empfehlenswert ist ein Bereich von 20 bis 0, insbesondere 10 bis 0, bevorzugt 5 bis 0°C unterhalb der Reaktionstemperatur.

Nachdem die Ausgangsstoffe auf die vorgegebene Temperatur erwärmt worden sind, führt man sie in Gegenwart des festangeordneten Katalysators zusammen. Alle drei Ausgangsstoffe Amin, Formaldehyd und Wasserstoff dürfen erst in Gegenwart des festangeordneten Katalysators aufeinandertreffen.

Wird der Wasserstoff zusammen mit einem der übrigen Ausgangsstoffe der Reaktion zugeleitet, so dürfen das Amin und der Formaldehyd erst in Anwesenheit des festangeordneten Katalysators zusammengeführt werden. Auch in diesem Falle geraten alle drei Ausgangsstoffe nur im Beisein des Kontaktes miteinander in Berührung. Technisch realisiert man diese Forderung dadurch, daß man die Leitung oder das Leitungssystem wenigstens eines der beiden Ausgangsstoffe Amin und Formaldehyd in der Katalysatorzone enden läßt. Somit wird sichergestellt, daß das Amin oder der Formaldehyd nur im Zugegensein von Katalysator austritt und mit den anderen Reaktionsteilnehmern zusammentrifft.

Nach einer bevorzugten Ausfuhrungsform verläßt der Formaldehyd die in der Katalysatorzone endende Leitung.

Es ist jedoch auch möglich, die Leitungen zweier oder auch aller drei Ausgangsstoffe in der Zone des festangeordneten Katalysators münden zu lassen. Welche Anordnung der Leitungen zu wählen ist, wird zum einen von den Stoffmengen und zum anderen von der Geometrie der Kontaktzone und der erforderlichen Strömungsverhältnissen abhängen. Bei Durchsatz größerer Stoffmengen je Zeiteinheit empfiehlt es sich, je Ausgangsstoff nicht nur eine, sondern mehrere Leitungen zu gebrauchen. Sollten die Strömungsverhältnisse eine ausreichende Durchmischung der Reaktanten in der Katalysatorzone nicht sicherstellen, ist die Verwendung zusätzlicher Verteilervorrichtungen nützlich. Diese Verteiler können beispielsweise als Ringbrause oder Duschkopf am Ende einer Leitung angebracht werden. Aber auch andere Verteilersysteme wie Düsen, Fritten oder Röhrenbündel können Verwendung finden.

Das erfindungsgemäße Verfahren besitzt gegenüber dem Stand der Technik einen entscheidenden Vorteil. Es ist nicht auf spezielle Katalysatoren beschränkt, sondern gestattet die Verwendung einer Vielzahl von festangeordneten Katalysatoren. Die bekannten Nachteile - Schädigung des Katalysators und Bildung unerwunschter Nebenprodukte - werden vermieden. Ein Zerfallen des Katalysators wird ebenso wie die Entstehung von Polymerprodukten wirksam unterbunden.

Die festangeordneten Katalysatoren können Trägerstoffe besitzen, aber auch frei von Trägern sein. Sie enthalten Ni, Co, Cu, Mn, Fe, Rh, Pd und/oder Pt insbesondere Ni, Co, Cu, Rh, Pd und/oder Pt bevorzugt Ni, Co und/oder Pd und daneben gegebenenfalls gebräuchliche Zusatzstoffe und Promotoren, beispielsweise Erdalkalioxide, $SiO_2$, $Al_2O_3$, $MnO_2$ und/oder $Cr_2O_3$.

Vorteilhaft ist der Gebrauch von Trägerkatalysatoren. Als Träger kommen $Al_2O_3$, $SiO_2$, Kieselerde, Aktivkohle, Bimsstein in Betracht.

4

EP 0 297 295 B1

Besonders günstig ist die Verwendung von Katalysatoren, mit 10 bis 75 insbesondere 20 bis 70, bevorzugt 40 bis 65 Gew.-% Ni, Co, Cu, Mn und/oder Fe bezogen auf gesamte Katalysatormasse.

Edelmetallkatalysatoren gestatten eine Umsetzung unter besonders milden Bedingungen. Sie sind üblicherweise auf einem Träger untergebracht und weisen einen Metallgehalt von 0,1 bis 20 insbesondere 0,2 bis 15, bevorzugt 0,5 bis 10 Gew.-% bezogen auf gesamte Katalysatormasse auf.

Geeignete Edelmetalle sind Rh, Pd und/oder Pt. Als Träger empfehlen sich geformte Materialien auf Basis von $Al_2O_3$, Aktivkohle, Kieselgel, Kieselgur und/oder Bimsstein.

Der Katalysator bestimmt die Reaktionsbedingungen, insbesondere die Reaktionstemperatur und den Druck.

Die Ni, Co, Cu, Mn und/oder Fe-haltigen Nichtedelmetallkatalysatoren erfordern 50 bis 250, insbesondere 70 bis 200, bevorzugt 100 bis 150°C und 3 bis 30, insbesondere 5 bis 20, bevorzugt 8 bis 15 MPa.

Edelmetallkatalysatoren benötigen 20 bis 165, insbesondere 30 bis 160, bevorzugt 50 bis 150°C und 0,1 bis 15, insbesondere 0,2 bis 12, bevorzugt 0,5 bis 10 MPa. Besonders schonende Bedingungen liefern Temperaturen bis 100°C. Eine schnellere Umsetzung erfolgt in einem Bereich von 100 bis 165, insbesondere 100 bis 160, bevorzugt 115 bis 150°C.

Das Verhältnis der am Aminstickstoff durch Methylgruppen zu ersetzenden Wasserstoffatome zu Formaldehyd beträgt 1:1 bis 1:2, insbesondere 1:1 bis 1:1,5, bevorzugt 1:1 bis 1:1,2. Je Mol Formaldehyd werden 1 bis 10, insbesondere 1,05 bis 5, bevorzugt 1,1 bis 2,5 Mol $H_2$ der Reaktion zugeleitet.

Überschüssig eingesetzter Wasserstoff kann in die Reaktion zurückgeführt werden.

Die Katalysatorbelastung (Volumen Einsatzgemisch / Schüttvolumen des Katalysators x Stunde = $V'/Vh$) beträgt 3 : 1 bis 0,2 : 1, insbesondere 2 : 1 bis 0,4 : 1, bevorzugt 1,5 : 1 bis 0,5 : 1.

Experimenteller Teil:

Der Reaktorbehälter besteht aus einem druckfesten Rohr mit einem Innendurchmesser von 28 mm. In den unteren Teil des Druckrohres werden Raschigringe, die einen Durchmesser von 3 mm aufweisen, bis zu einer Höhe von 800 mm eingefüllt. Diese Zone dient als Vorheizzone, in der die Ausgangsstoffe auf die vorgegebene Temperatur aufgeheizt werden. Die Beheizung erfolgt über einen den Reaktor umgebenden Heizmantel. Oberhalb der Vorheizzone befindet sich der festangeordnete Katalysator.

Der Reaktor wird zu Beginn der Umsetzung mit Amin befüllt.

Dann wird weiteres Amin zusammen mit Wasserstoff von unten in den Reaktor eingebracht und durchströmt die mit Raschigringen befüllte Vorheizzone, während der Formaldehyd über ein separates Steigrohr, das durch die Vorheizzone führt, direkt in die Schicht des festangeordneten Katalysators gepumpt und erst dort mit dem Amin und dem Wasserstoff vermischt wird. Die Vorheizzone wird mittels des den Reaktor außen umgebenden Heizmantels erhitzt und die Ausgangsstoffe werden auf die gewünschte vorgegebene Temperatur gebracht. Nach Verlassen der Katalysatorzone wird das Reaktionsgemisch am Kopf des Reaktors abgezogen und einem Druckabscheider zugeführt.

Beispiel 1

Kontinuierliche Herstellung von N,N-Dimethyl-n-octylamin

In den vorstehend beschriebenen Reaktorbehälter werden 300 ml eines tablettierten Ni-Katalysators (~50 bis 53 Gew.-% Ni und etwa 25 bis 30 Gew.-% Kieselgur als Träger; Handelsprodukt der Hoechst AG: RCH Ni 52/35) eingefüllt.

Der Reaktor wird mit n-Octylamin gefüllt. Anschließend werden bei 120°C und 10 MPa $H_2$-Druck 60 ml n-Octylamin/Stunde ($V'/Vh$ = 0,2) bzw. 120 ml n-Octylamin/Stunde ($V'/Vh$ = 0,4) und überschüssiger Wasserstoff (200 $1H_2$/Stunde) eingesetzt.

Formaldehyd (~55 Gew.-% Formaldehyd, ~ 35 Gew.-% Methanol und ~10 Gew.-% Wasser) wird in einem Überschuß von 10 Mol.-% bezogen auf Amin zugeleitet.

Das entstehende Reaktionsgemisch wird über Kopf des Reaktorbehälters abgezogen und in dem nachgeschalteten Druckabscheider von überschüssigem Wasserstoff abgetrennt.

Die erzielten Resultate sind in Tabelle 1 zu entnehmen. Die gaschromatografisch ermittelten Werte beziehen sich auf wasserfreies Produkt.

5

## Tabelle 1

### Produktzusammensetzung

|  | V'/Vh = 0,2* | V'/Vh = 0,4* |
|---|---|---|
| Methanol | 28,4 | 28,5 |
| N-Methyl-n-octylamin | 2,5 | 3,6 |
| N,N-Dimethyl-n-octylamin | 68,9 | 66,7 |
| Höhersieder | 0,2 | 1,2 |

*) bezogen auf n-Octylamin-Einsatz

Beispiel 2

Kontinuierliche Herstellung von N,N-Dimethylanilin

Es wird wie in Beispiel 1 gearbeitet (gleiche Apparatur; gleicher Katalysator).

Der Reaktor wird mit Anilin gefüllt. Anschließend werden bei 110°C und 10 MPa $H_2$-Druck 90 ml Anilin/Stunde (V'/Vh = 0,3) und überschüssiger Wasserstoff (200 l$H_2$/Stunde) eingesetzt.

Formaldehyd wird wie in Beispiel 1 angegeben zugeleitet.

Das Reaktionsgemisch fällt in zwei Phasen an. Die obere enthält nahezu das gesamte Wasser und den größten Teil des Methanols. Die untere Schicht besteht aus dem Wertprodukt.

Die erzielten Resultate (Gew.-% bezogen auf wasserfreies Produkt) sind der nachfolgenden Tabelle 2 zu entnehmen.

Tabelle 2

| Produktzusammensetzung (untere Phase): | |
|---|---|
| Methanol | 7,5 % |
| Zwischenlauf | 5,5 % |
| Anilin | 1,2 % |
| N,N-Dimethyl-anilin | 75,8 % |
| Höhersieder | 10,0 % |
| Wassergehalt | 0,7 %. |

Beispiel 3

Kontinuierliche Herstellung von N,N-Dimethylpiperazin

Es wird wie in Beispiel 1 gearbeitet (gleiche Apparatur, gleicher Katalysator).

Der Reaktor wird mit Piperazin gefüllt. Anschließend werden bei 110°C und 10 MPa $H_2$-Druck 215 ml Piperazinlösung/Stunde (Volumverhältnis Piperazin: $CH_3OH$ = 1 : 1) und überschüssiger Wasserstoff (200 l$H_2$/Stunde) eingesetzt.

Formaldehyd wird wie in Beispiel 1 angegeben zugeleitet.

Das erzielte Resultat (Gew.-% bezogen auf wasserfreies Produkt) ist Tabelle 3 zu entnehmen.

Tabelle 3

| Produktzusammensetzung | |
|---|---|
| Methanol | 37,1 |
| Zwischenlauf | 0,1 |
| N,N′-Dimethyl-piperazin | 62,2 |
| N-Methyl-piperazin | 0,1 |
| Piperazin | 0,3 |
| Höhersieder | 0,2 |

N,N′-Dimethyl-piperazin kann hieraus in ≧ 99 %iger Reinheit destillativ erhalten werden.

Beispiel 4

Kontinuierliche Herstellung von N,N-Dimethyldiglykolamin

Es wird wie in Beispiel 1 gearbeitet (gleiche Aparatur, gleicher Katalysator).
Der Reaktor wird mit Diglykolamin gefüllt. Anschließend werden bei 120°C und 10 MPa $H_2$-Druck 100 ml Diglykolamin/-Stunde (V′/Vh = 0,33) bzw. 150 ml Diglykolamin/Stunde (V′/Vh = 0,5) eingesetzt.
Formaldehyd wird wie in Beispiel 1 angegeben zugeleitet.
Die erzielten Resultate (Gew.-%, bezogen auf wasserhaltiges Produkt) sind Tabelle 4 zu entnehmen.

Tabelle 4

| V′/Vh | 0,33 | 0,5 | 0,5 |
|---|---|---|---|
| Produktzusammensetzung | | | |
| Vorlauf | <0,1 | 0,1 | 0,1 |
| Methanol | 29,9 | 29,2 | 28,5 |
| Zwischenlauf | 0,4 | 0,3 | 0,4 |
| N,N-Dimethyldiglykolamin | 49,8 | 49,8 | 49,8 |
| Nachlauf | 0,2 | 0,3 | 0,4 |
| $H_2O$ | 19,7 | 20,3 | 20,8 |

Beispiele 5 bis 12

Kontinuierliche Herstellung von N,N-Dimethyl-n-octylamin

Es wird wie in Beispiel 1 (gleiche Apparatur, aber andere Katalysatoren) gearbeitet.
Als Katalysator werden je 300 ml eines Palladium-Katalysators (5 Gew.-% Pd auf Aluminiumoxid-Träger in tablettierter Form; Handelsprodukt der Degussa: Typ E 263P), eines Kobaltkatalysators (43 bis 46 Gew.-% Co, Kieselgur als Träger; Handelsprodukt der Hoechst AG: RCH Co 45/20), eines Kupfer-Katalysators (etwa 60 Gew.-% Cu, $SiO_2$ als Träger; Handelsprodukt der Hoechst AG: RCH Cu 60/35) und ein Platin-Katalysator (5 Gew.-% Pt auf gekörnter Aktivkohle) verwendet.
Der Reaktor wird mit n-Octylamin gefüllt. Anschließend werden bei 120°C und 5 oder 10 MPa $H_2$-Druck 90 ml oder 75 ml n-Octylamin/Stunde (V′/Vh = 0,3 oder 0,25) und überschüssiger Wasserstoff (200 l$H_2$/Stunde) eingesetzt.
Formaldehyd wird wie in Beispiel 1 angegeben zugeleitet.
Reaktionsbedingungen und Produktzusammensetzung (Gew.-% bezogen auf wasserfreies Produkt) sind der nachfolgenden Tabelle 5 zu entnehmen.

**Tabelle 5**

| Beispiel | 5 | 6 | 7 | 8 | 9 | 10 | 11 | 12 |
|---|---|---|---|---|---|---|---|---|
| Katalysator | Degussa E263P | | RCH Co 45/20 | | RCH Cu 60/35 | | Pt auf gekörnter Aktivkohle | |
| Reaktionstemperatur (°C) | 120 | | 120 | | 120 | | 120 | |
| Druck (MPa $H_2$) | 10 | 5 | 10 | 5 | 10 | 5 | 5 | 10 |
| V'/Vh | 0,30 | | 0,25 | | 0,25 | | 0,25 | |
| **Produktzusammensetzung** | | | | | | | | |
| Methanol | 21,8 | 20,9 | 33,7 | 37,6 | 31,3 | 23,1 | 19,1 | 21,7 |
| Zwischenlauf | 2,3 | 1,5 | 2,1 | 1,2 | 1,7 | 1,7 | 3,7 | 3,4 |
| N,N-Dimethyl-n-octylamin | 75,7 | 77,6 | 59,6 | 56,0 | 66,4 | 74,2 | 76,8 | 73,9 |
| Zwischenlauf | – | – | – | – | 0,4 | 1,0 | 0,4 | 0,5 |
| N,N-Dioctyl-methylamin | 0,2 | – | 4,4 | 5,2 | – | – | – | 0,5 |
| Höhersieder | – | – | 0,2 | – | 0,2 | – | – | – |

**Patentansprüche**

1. Verfahren zur Herstellung von Methylaminen durch Umsetzung von Aminen, Formaldehyd und Wasserstoff als Ausgangsstoffe unter Druck und erhöhter Temperatur an einem festangeordneten Katalysator in flüssiger Phase, dadurch gekennzeichnet, daß der Formaldehyd 0 bis 50 Gew.-% Wasser enthält, die Ausgangsstoffe voneinander getrennt auf eine vorgegebene Temperatur erhitzt und anschließend in Gegenwart des festangeordneten Katalysators unter Vermischung zusammengeführt werden.

2. Verfahren nach Anspruch 1, dadurch gekennzeichnet, daß der Druck 0,1 bis 30, insbesondere 1 bis 20, bevorzugt 2 bis 15 MPa und die Temperatur 20 bis 250, insbesondere 50 bis 200, bevorzugt 70 bis 150°C beträgt.

3. Verfahren nach Anspruch 1 oder 2, dadurch gekennzeichnet, daß Amin und Formaldehyd voneinander getrennt auf die vorgegebene Temperatur erhitzt werden.

4. Verfahren nach einem oder mehreren der Ansprüche 1 bis 3, dadurch gekennzeichnet, daß als Amine organische Verbindungen mit einer oder mehreren primären und/oder sekundären Amingruppen verwendet werden.

5. Verfahren nach einem oder mehreren der Ansprüche 1 bis 4, dadurch gekennzeichnet, daß als Amine einwertige und/oder mehrwertige primäre und/oder sekundäre Amine verwendet werden.

6. Verfahren nach einem oder mehreren der Ansprüche 1 bis 5, dadurch gekennzeichnet, daß als Amine aliphatische, cycloaliphatische, araliphatische, aromatische und heterocyclische Amine verwendet werden.

7. Verfahren nach einem oder mehreren der Ansprüche 1 bis 6, dadurch gekennzeichnet, daß der Formaldehyd in gelöster Form eingesetzt wird und die Formaldehydlösung 5 bis 30, bevorzugt 7 bis 15 Gew.-% Wasser enthält.

8. Verfahren nach einem oder mehreren der Ansprüche 1 bis 7, dadurch gekennzeichnet, daß die vorgegebene Temperatur nicht mehr als 20, insbesondere 10 bevorzugt 5°C unterhalb der Reaktionstemperatur und nicht mehr als 10, insbesondere 5, bevorzugt 0°C oberhalb der Reaktionstemperatur liegt.

9. Verfahren nach einem oder mehreren der Ansprüche 1 bis 8, dadurch gekennzeichnet, daß die vorgegebene Temperatur 20 bis 0, insbesondere 10 bis 0, bevorzugt 5 bis 0°C unterhalb der Reaktionstemperatur liegt.

10. Verfahren nach einem oder mehreren der Ansprüche 1 bis 9, dadurch gekennzeichnet, daß der Katalysator ein Trägerkatalysator oder ein trägerfreier Metallkatalysator ist.

11. Verfahren nach einem oder mehreren der Ansprüche 1 bis 10, dadurch gekennzeichnet, daß der Katalysator Ni, Co, Cu, Mn, Fe, Rh, Pd und/oder Pt und daneben gegebenenfalls gebräuchliche Zusatzstoffe und Promotoren enthält.

12. Verfahren nach einem oder mehreren der Ansprüche 1 bis 11, dadurch gekennzeichnet, daß der Katalysator als Träger $Al_2O_3$, $SiO_2$, Kieselerde oder Aktivkohle enthält.

13. Verfahren nach einem oder mehreren der Ansprüche 1 bis 12, dadurch gekennzeichnet, daß als Zusatzstoffe und Promotoren im Katalysator Erdalkalioxide, $SiO_2$, $Al_2O_3$, $MnO_2$ und/oder $Cr_2O_3$ enthalten sind.

## Claims

1. A process for the preparation of methylamines by the reaction of amines, formaldehyde and hydrogen as starting materials under pressure and at elevated temperature on a fixed-bed catalyst in the liquid phase, characterised in that the formaldehyde contains 0 to 50% by weight of water, the starting materials are heated separately to a given temperature and subsequently mixed with each other in the presence of the fixed-bed catalyst.

2. A process according to claim 1, characterised in that the pressure is 0.1 to 30, in particular 1 to 20 and preferably 2 to 15 MPa and the temperature is 20 to 250, in particular 50 to 200 and preferably 70 to

EP 0 297 295 B1

150°C.

3. A process according to claim 1 or 2, characterised in that the amine and the formaldehyde are separately heated to the given temperature.

4. A process according to one or more of the claims 1 to 3, characterised in that organic compounds having one or several primary and/or secondary amine groups are used as amines.

5. A process according to one or more of the claims 1 to 4, characterised in that monovalent and/or multivalent primary and/or secondary amines are used as amines.

6. A process according to one or more of the claims 1 to 5, characterised in that aliphatic, cycloaliphatic, araliphatic, aromatic and heterocyclic amines are used as amines,

7. A process according to one or more of the claims 1 to 6, characterised in that the formaldehyde is used in dissolved form and that the formaldehyde solution contains 5 to 30, preferably 7 to 15% by weight of water.

8. A process according to one or more of the claims 1 to 7, characterised in that the given temperature is not more than 20, in particular 10 and preferably 5°C below the reaction temperature and not more than 10°C, in particular 5 and preferably 0°C above the reaction temperature.

9. A process according to one or more of the claims 1 to 8, characterised in that the given temperature is 20 to 0, in particular 10 to 0 and preferably 5 to 0°C below the reaction temperature.

10. A process according to one or more the claims 1 to 9, characterised in that the catalyst is a support catalyst or a support-free metal catalyst.

11. A process according to one or more of the claims 1 to 10, characterised in that the catalyst contains Ni, Co, Cu, Mn, Fe, Rh, Pd and/or Pt and, in addition, in some cases common additives and promoters.

12. A process according to one or more of the claims 1 to 11, characterised in that the catalyst contains $Al_2O_3$, $SiO_2$, siliceous earth or activated carbon as a support.

13. A process according to one or more of the claims 1 to 12, characterised in that alkaline earth oxides, SiO2, $Al_2O_3$, $MnO_2$ and/or $Cr_2O_3$ are contained in the catalyst as additives and promoters.

**Revendications**

1. Procédé pour la préparation de méthylamines par réaction d'amines, du formaldéhyde et de l'hydrogène (produits de départ) sous pression et à température élevée sur un catalyseur en lit fixe, en phase liquide, caractérisé en ce que le formaldéhyde contient de 0 à 50 % en poids d'eau, les produits de départ sont chauffés séparément les uns des autres à une température déterminée puis envoyés et mélangés en présence du catalyseur en lit fixe.

2. Procédé selon la revendication 1, caractérisé en ce que la pression est de 0,1 à 30, plus spécialement de 1 à 20, de préférence de 2 à 15 MPa et la température de 20 à 250, plus spécialement de 50 à 200 et de préférence de 70 à 150°C.

3. Procédé selon la revendication 1 ou 2, caractérisé en ce que l'on réchauffe l'amine et le formaldéhyde, séparément, à la température voulue.

4. Procédé selon une ou plusieurs des revendications 1 à 3, caractérisé en ce que les amines mises en oeuvre sont des composés organiques contenant un ou plusieurs groupes amino primaires et/ou secondaires.

5. Procédé selon une ou plusieurs des revendications 1 à 4, caractérisé en ce que les amines mises en oeuvre sont des amines primaires et/ou secondaires monovalentes et/ou polyvalentes.

10

**6.** Procédé selon une ou plusieurs des revendications 1 à 5, caractérisé en ce que les amines mises en oeuvre sont des amines aliphatiques, cycloaliphatiques, araliphatiques, aromatiques et hétérocycliques.

**7.** Procédé selon une ou plusieurs des revendications 1 à 6, caractérisé en ce que le formaldéhyde est mis en oeuvre à l'état de solution et en ce que la solution de formaldéhyde contient de 5 à 30, de préférence de 7 à 15 % en poids d'eau.

**8.** Procédé selon une ou plusieurs des revendications 1 à 7, caractérisé en ce que la température déterminée en question se situe à 20°C au maximum, plus spécialement à 10°C et de préférence à 5°C au maximum au-dessous de la température de réaction et à 10°C au maximum, plus spécialement à 5°C et de préférence à 0°C au-dessus de la température de réaction.

**9.** Procédé selon une ou plusieurs des revendications 1 à 8, caractérisé en ce que la température déterminée en question se situe à un niveau de 20 à 0, plus spécialement de 10 à 0 et de préférence de 5 à 0°C au-dessous de la température de réaction.

**10.** Procédé selon une ou plusieurs des revendications 1 à 9, caractérisé en ce que le catalyseur est un catalyseur sur support ou un catalyseur métallique sans support.

**11.** Procédé selon une ou plusieurs des revendications 1 à 10, caractérisé en ce que le catalyseur contient Ni, Co, Cu, Mn Fe, Rh, Pd et/ou Pt et, avec ces métaux, le cas échéant, des additifs et activateurs usuels.

**12.** Procédé selon une ou plusieurs des revendications 1 à 11, caractérisé en ce que le catalyseur contient en tant que support $Al_2O_3$, $SiO_2$ ou du charbon actif.

**13.** Procédé selon une ou plusieurs des revendications 1 à 12, caractérisé en ce que les additifs et activateurs contenus dans le catalyseur sont des oxydes alcalino-terreux, $SiO_2$, $Al_2O_3$, $MnO_2$ et/ou $Cr_2O_3$.